# EUROPEAN PATENT APPLICATION

(11) **EP 2 338 475 A2**
(43) Date of publication of application: **29.06.2011**
(21) Application number: 10192668.1
(22) Date of filing: 05.11.2002
(51) Int. Cl.: A61K 9/00, A61P 1/02

(54) **Treating canker sores with patches to speed healing and relieve pain**

(30) Priority: 05.11.2001 US 332916 P; 28.12.2001 US 344577 P; 04.09.2002 US 236289
(62) Divisional of application: 02791205.4
(71) Applicant: Orahealth Corporation, Bellevue, WA 98004 (US)
(72) Inventor: HALEY, Jeffrey T., Mercer Island, WA 98040-4440 (US)
(74) Representative: Maccalli, Marco

(57) **Abstract**

A method for treating canker sores with oral patches to speed healing and relieve pain. If certain medications are applied to a canker sore using an oral patch that restricts local flow of saliva and delivers the medication for at least 30 minutes and the patches are used for at least two or more hours per day, the method reduces the healing time for canker sores from typical 10 - 14 days to 2 days. The method can be used with various antimicrobials that speed the healing of canker sores, including penicillin and amoxicillin. The method can also be used with amlexanox and de-glycyrrhizinated licorice extract (DGL) which work by an unknown method. When used with DGL, the method also quickly relives canker sore pain and, if used before commencing a meal, typically relieves pain through a complete meal. A soluble oral patch with DGL is disclosed.

## Description

### BACKGROUND

To deliver a medication in the mouth over time for treatment of health problems in the mouth or throat, oral patches have been developed. An oral patch typically includes one or more flexible layers that do not dissolve entirely such as invented by Anthony et al. and disclosed in US patent 5,713,852. Another example of an oral patch is the DentiPatch which has one or more non-soluble thermo-plastic layers and lidocaine, offered for sale by Noven Pharmaceuticals, Inc.

As used herein, the word "patch" does not include preparations that move about the mouth rather than resting in one place, such as cough drops or throat lozenges, and therefore do not impede local flow of saliva. Nor does it include preparations that do not hold together as a single item when held in the mouth such as preparations of powder, liquid, paste, viscous liquid gel, or a tablet or troche that crumbles into a powder or paste when chewed or placed in saliva. Conversely, it does include a preparation formed of a gelled hydrocolloid that holds together as a single item when held in the mouth, such as the soft, adherent, soluble oral patch disclosed by the same inventor in US patent application serial number _________________ filed November 5, 2002, which is incorporated herein by this reference.

The most significant differences between an oral patch as used herein and other forms of medicinal preparations is that an oral patch is designed to release medication into the mouth over a relatively long period of time, such as 30 minutes or more, restrict local flow of saliva so that the medication can reach high concentrations along side the patch, and remain in the mouth as a single item that will not spread to be in a plurality of locations in the mouth at one time.

It has been known for many decades that licorice root includes an ingredient that speeds the healing of ulcers in the stomach. It is not yet known which ingredient in licorice root is responsible. However, it is not the glycyrrhizic acid or the glycyrrhetenic acid, which are known to cause significant physiologic effects, including an increase in blood pressure. When these acids are removed, such as by a process described in US Patent No. 3,046,195, the resulting de-glycyrrhizinated licorice (DGL) is still effective for treatment of ulcers in the stomach. More recently it has been discovered that a majority of stomach ulcers are caused by bacteria called *Helicobacter pylori* and that most stomach ulcers are therefore treatable with pharmaceutical antibiotics. This suggests that the active ingredient in DGL is an antimicrobial that interferes with the *Helicobacter pylori* bacteria or with a reaction of stomach tissues to the bacteria.

For treatment of ulcers in the stomach, powdered DGL is sometimes swallowed in gelatin capsules. However, tests have shown that the DGL is more effective if it is mixed with saliva before being swallowed. Therefore, chewable DGL tablets and lozenges have been developed. DGL powder is compressed into a tablet or lozenge form that quickly crushes into a mass of powder or paste when chewed. Even if it is not chewed, when exposed to saliva, it quickly dissolves into a mass of paste and mixes with saliva. All of these DGL tablets and lozenges are designed to be chewed and the resulting paste swallowed. It is not known to put DGL in an oral patch.

Physicians in India have determined with clinical trials that DGL is also effective for speeding the healing of canker sores (aphthous ulcers) when DGL in water is swished in the mouth several times per day. Das SK, Gulati AK, Singh VP; De-glycyrrhizinated Licorice in Aphthous Ulcers; J Assoc Physicians India, 1989; 37:647. It also quickly relieves pain from canker sores without numbing surrounding tissues.

Other physicians (Weil) have reported success in speeding the healing of canker sores using propolis, made by bees, which is believed to have antimicrobial effects. A drug recently approved by the FDA for use to speed recovery from canker sores, amlexanox, works by an unknown method.

As reported in standard medical references, topical application of pharmaceutical antibiotics such as tetracycline or penicillin have been shown to speed healing of canker sores (aphthous ulcers) when swished as a liquid in the mouth several times per day. Alternatively, antibiotics may be applied to the canker sore several times per day as a powder, paste, or viscous liquid gel or they may be placed into the mouth as a tablet or troche which quickly dissolves into a liquid or paste, as disclosed by Hau in US patent number 6,248,718, the contents of which are incorporated herein by this reference. In addition to tetracycline and penicillin, other antibiotics such as amoxicillin have been shown effective when applied topically in a similar manner. Other products that have been reported to speed healing of canker sores when applied topically include antibacterials such as hydrogen peroxide, carbamide peroxide, chlorhexidine gluconate and, in at least one study, the antihistamine diphenhydramine.

Anti-inflammatory drugs, such as corticosteroids, reduce severity of canker sores but do not speed healing.

### SUMMARY OF THE INVENTION

Through trials, the inventor has discovered that, when using amoxicillin pharmaceutical antibiotic or DGL herbal extract for treatment of canker sores (aphthous ulcers), it is better to block local flow of saliva and keep the medication in the mouth on or near the sore as many hours per day as possible through the use of an oral patch. Using a patch to keep the medication topically applied to the canker sore for about half of the hours in a day over 2 days, instead of merely applying a troche, gel, paste, or liquid six times per day for 2 days, transforms the results from nearly always ineffective to nearly always effective. With prompt and consistent use on each canker sore, the duration of each canker sore is reduced from typically 10-14 days without treatment to typically 2 days with DGL or amoxicillin in a patch applied for half of the hours, day and night. For small or emergent canker sores, application for as little as two hours per day can be effective. Thus, healing of canker sores is accelerated by use of an oral patch with an effective antimicrobial, which term includes and is not limited to agents that might be described as antibiotics, antiprotoctistas, antibacterials, or antiarchaeals.

An explanatory theory is that canker sores are triggered by a reaction of the mouth tissues to either a surface of a common microbe, such as a bacterium, or to a chemical excreted by the microbe, such as when the microbe is allowed to the tissues where the mucous membrane is disrupted, such as by a small cut. People whose mouth tissues are sensitive to the microbe or its excreta or whose immune systems do not adequately defend against the microbe will suffer from canker sores while a majority of the population will not. An inflammatory reaction is involved. In addition, there may be an allergic (histaminic) reaction or an autoimmune reaction, which may or may not be synergistic with the inflammatory reaction.

This theory is supported by observation that all of the agents mentioned above that have been shown to speed the healing of canker sores are "antimicrobials" as that term is used herein, except for amlexanox, which might also be an antimicrobial though its action is unknown, and the antihistamine. However, presuming that the action of the antihistamine is to suppress histaminic reaction to a microbe or its excreta in contact with mouth tissues at a disruption in the mucous membrane, even the antihistamine may be classed as an "antimicrobial" as used herein. By "antimicrobial" the inventor means an agent that stops the tissue reaction to the microbe whether this is accomplished by: (1) disrupting the microbes in the ulcer, (2) neutralizing the microbe's excreta, (3) interfering with the tissue's reaction to the microbe or its excreta, (4) inhibiting the action of an enzyme that is active because of the microbe's presence, (5) any other process, or (6) a combination of the above.

This theory is further supported by observations that: (1) both canker sores and stomach ulcers are ulcerations of parts of the mucous membranes of the gastro-intestinal tract which share many similarities; (2) DGL has been shown effective for speeding healing of both canker sores and stomach ulcers; and (3) stomach ulcers have been shown to be caused by a bacterium. These observations suggest that DGL acts to disrupt microbe-caused problems in both stomach ulcers and canker sores and, in that sense, the active ingredient in DGL, whatever it may be, is an "antimicrobial" as used herein.

This theory is consistent with the inventor's observations that holding DGL or amoxicillin on an aphthous ulcer for many hours per day with an oral patch that impedes local flow of saliva, even if the agent does not have sufficient concentration to kill or significantly disrupt microbes in other parts of the mouth or elsewhere in the body, is effective.

It may be that a relatively minor disruption or modification of microbial colonies within the ulcer, without significantly disrupting the same microbes elsewhere in the mouth, are sufficient to allow the ulcer to heal. The inventor has discovered that this disruption must be maintained over a substantial portion of each day through the use of a patch that blocks local flow of saliva and releases medication over time to achieve high rates of effectiveness.

The inventor also discovered that when extract of licorice root is held on a canker sore with an oral patch for longer than 15 minutes, the canker sore pain disappears and there is no numbing of surrounding tissues. De-glycyrrhizinated extract of licorice root (DGL) is preferred to avoid side effects of glycyrrhizin, and this form of licorice root extract works as well to relieve pain. The pain relief continues while eating long enough to complete a meal without pain.

In one aspect, the invention is a method for treating canker sores by providing patches which, when exposed to saliva in a human mouth, release a medication over more than 30 minutes, the medication being an agent that speeds healing of canker sores, and instructing people to hold the patches in their mouths for at least 2 or more hours per day to treat canker sores. The medication may be an extract of licorice root such as DGL. The medication may be an antimicrobial such as a pharmaceutical antibiotic or an antibacterial. The medication may be amlexanox. The medication may be propolis or an extract of propolis. The patch may include a binder ingredient to hold and release the medication. The binder ingredient may be a gum that dissolves in saliva such as xanthan gum, konjac gum, gelatin, or locust bean gum.

In another aspect, the invention is a method for treating canker sores to relieve pain of canker sores without numbing surrounding tissues by providing a patch which, when exposed to saliva in the human mouth, releases such a medication over more than 30 minutes and instructing recipients of the patches to use them on or near canker sores to relieve pain. The medication may be an extract of licorice root, such as DGL. The patch may include a binder ingredient that dissolves in saliva, such as xanthan gum, konjac gum, gelatin, or locust bean gum.

In another aspect, the invention is an oral patch that, when held in a human mouth, remains in the mouth as a single item that will not spread to be in a plurality of locations in the mouth at one time and releases DGL extract of licorice root. The release of DGL may last more than 30 minutes. The patch may include a binder ingredient, such as a gum that dissolves in saliva, such as gum arabic, carrageenan, xanthan gum, konjac gum, agar, gelatin, or locust bean gum.

### BRIEF DESCRIPTION OF THE DRAWINGS

Figure 1a shows a side view of an oral patch that completely dissolves.
Figure 1b shows a top view of the same oral patch.
Figure 2 shows a more conventional non-dissolving oral patch covering a canker sore.

### DETAILED DESCRIPTION

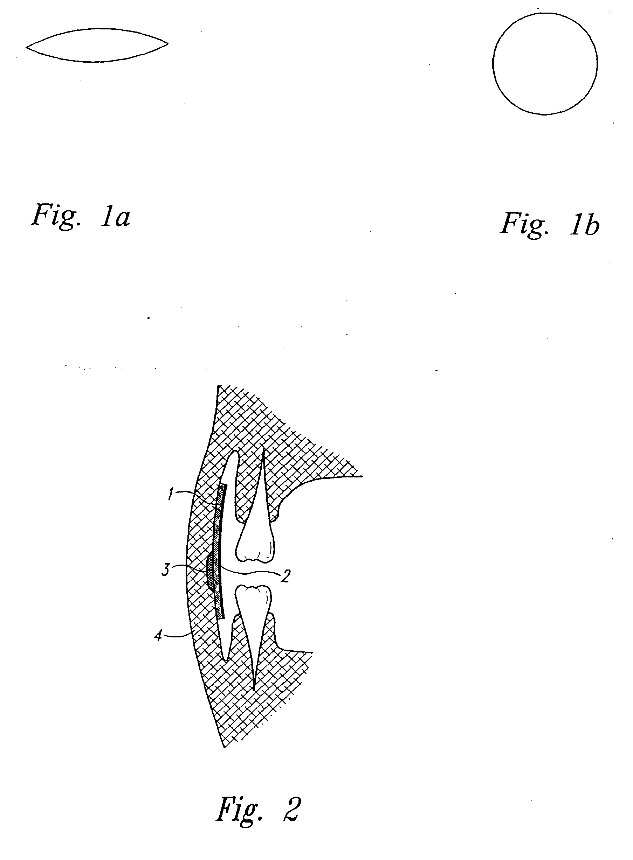

Figure 1 shows an oral patch that completely dissolves. It has a feel and texture like gummy candies. It is made with slowly dissolving hydrocolloids so that that it typically lasts in the mouth for at least one to six hours. The patch can be formed in the shape of a tablet or a lozenge or a wafer or any other desired shape. The preferred shape is a thin lentil as shown in Figure 1a. A detailed description of the patch and how to make it are disclosed by the same inventor in US patent application serial number filed November 5, 2002 which is incorporated into this document by reference. ,

To cause the patch to dissolve very slowly in saliva, a binder that dissolves slowly in saliva is incorporated. Binders that have been tested and found to work well include carrageenan (preferably kappa), xanthan gum combined with konjac gum, and agar. Another useable gum is gum arabic. Other gums similar to those listed, such as locust bean gum which has properties similar to konjac gum, and guar gum should also work.

In addition to causing the patch to dissolve very slowly in the mouth, the binder also moderates any strong flavors by spreading out over a long period of time the release of that flavor. Consequently, sweeteners and other products to mask strong flavors are not required, although some users prefer a small amount of sweetener and some also prefer the addition of anise or other flavors.

The preferred method of manufacturing the patches of Figure 1 is to use gum drop manufacturing equipment, squirting a hydrated mixture heated above the gel melting temperature through nozzles onto a cornstarch mold, allowing the patches to cool and gel, drying the patches, and releasing them from the molds. The patches are preferably dried until the water activity level is lower than 0.8 so that the patches will not grow mold or other organisms. The patches are packaged with a hermetic seal to prevent absorption of water moisture from air.

Alternatively, instead of depositing hydrated mixture onto cornstarch molds, the mixture is deposited as an array of hot, viscous drops onto a sheet of high temperature plastic or coated paper. The drops are allowed to cool and then the sheets of plastic or coated paper with the drops on them are dried in a drying room or oven till the water activity level less than 0.8. The product is sold still adhered to the plastic or paper and the user pulls it off the plastic or paper.

Figure 2 shows a more conventional non-dissolving oral patch comprising a permeable sponge layer **1** and a non-permeable smooth outer layer **2**. The oral patch is covering a canker sore **3** in a human cheek **4**. The sponge layer **1** may be fibrous such that small fibers protrude from the surface and engage the mucous lining of the cheek. The fibers may comprise a non-woven mat such as a mat of cotton or synthetic, preferably a hydrophilic synthetic. The outer layer **2** is preferably smooth to minimize dislodging of the patch and may be formed of any flexible thermoplastic. Medication is held in the sponge **1** either by using a high viscosity liquid medication that slowly oozes out of the sponge or by binding the medication to the sponge with slowly dissolving binders such as any of the gums described above, including gelatin. A hot mixture of gums dissolved in water may be massaged into the sponge and then allowed to gel by cooling. A preferred size for the patch is 24 millimeters by 18 millimeters, and one or both layers of the patch may include a red pigment to color it like the inside of the mouth.

Alternatively, any of the other oral patches known in the art may be used.

For medication to be placed in the patch, any of the above-mentioned antimicrobials, or similar antimicrobials, may be used. Using DGL presents significant advantages over pharmaceutical antibiotics because, in the quantities that a person could use per day, no side affects have been discovered. The preferred quantity of DGL in each patch that lasts 1 to 6 hours is 95 milligrams. A person would have to consume more than 10 oral patches to achieve a dose of one gram of DGL, which is still a quite moderate dose for treatment of stomach ulcers. Nevertheless, because the DGL is concentrated on the canker sore and local flow of saliva is blocked by the patch, the treatment is effective for speeding the healing of the canker sores.

Presented below are conclusions from testing of the soft, adherent, soluble oral patches with 95 mg of DGL:
**Stinging sensation:** For the first 10 - 15 minutes after application of DGL, an active canker sore will sting. Then the stinging will end and the canker sore will no longer be painful. If there is no stinging when the DGL patch is applied and it has been more than 4 hours since DGL was on the sore, either there is no canker sore or it has begun to heal.
**Pain relief:** Using a DGL patch for 10-15 minutes before a meal relieves pain of the canker sore, and, if used up to commencement of a meal, the pain relief lasts through a typical meal. There is no numbing effect on surrounding tissues.
**How many to use in a day:** The more hours per day the better. Users who report using 2 or fewer DGL patches per day on an established canker sore, each soluble patch lasting 1 - 4 hours, often report that it does not work. Users who report using 4-9 patches per day on an established canker sore usually report success. When a sore has just begun and has not yet grown, one to four patches will usually do the job.
**How long to continue using the DGL patches:** We do not yet know. It may be that use can be discontinued use once the user applies DGL and has no stinging sensation and it has been more than 4 hours since DGL was last on the sore. There are reports of people discontinuing use once the sore was no longer painful and the sore then returned. Everyone who continued to use for 24 hours after the sore was no longer painful reported that the sore did not return.
**Catching it early:** If the user catches the canker sore early, shorter treatment is required. The sore will often start in a small cut. Some users report that if they apply one DGL patch to a cut for 1-4 hours before there is any sensation of a canker sore, then they will not get a canker sore from the cut. Other times, the sore starts with a feeling that the mucous layer is becoming too thin in a spot before it becomes painful. Some users report that if they apply one DGL patch to that spot, no canker sore develops. Some users report that if they begin applying the patch when the canker sore is very small and barely painful, only 24 hours of treatment is required, but if they wait until the sore is as large as a tomato seed, then they need 48 hours of treatment before it starts to heal.
**Treatment of the tongue:** For treatment of the tongue, most users stick a DGL patch that releases DGL on both sides to the closest tooth. This works particularly well at night.
**Braces:** Users with braces apply the patch to the braces opposite the canker sore so that the patch is touching the canker sore most of the time and is stuck to the teeth and braces. As it softens, the patch settles into the braces. It will completely dissolve out of the braces in 3 - 9 hours. All this time it supplies DGL medication to the sore.

Because use of antibiotics, antibacterials, and other pharmaceutical antimicrobials causes more adverse side affects than DGL, experimentation with these medications has been limited. However, sufficient experimentation was done with amoxicillin on conventional patches to determine that it works quite well. Conventional amoxicillin powder for preparation of oral suspension was used, which includes sugars and other excipients. The sugars act as a binder to the sponge of the oral patch. The oral suspension is massaged into the patch and dried. Preferably, the amoxicillin powder is mixed into a stronger than normal liquid before being added to the patch.

While particular embodiments of the invention have been described above the scope of the invention should not be limited by the above descriptions but rather limited only by the following claims.

## Claims

1. A lenticular shaped oral adhering disc, comprising:
(a) a circumferential edge and two sides at least one of which sides is a convex surface, the disc having a thickness at its center greater than a thickness near the edge, and
(b) one or more binder ingredients that adheres to mucosa and slowly dissolves in saliva when held in a human mouth
(c) wherein the binder ingredients which adhere to mucosa are in a sufficiently high ratio to other ingredients which reduce adhesion to mucosa that the disc effectively adheres to mucosa in the mouth.

2. The disc of claim 1 wherein, when placed in a human mouth and not chewed, the disc does not disintegrate into more than one piece.

3. The disc of claims 1 or 2 wherein, when placed in a human mouth and not chewed, the disc lasts for more than one hour before completely dissolving.

4. The disc of any one of the preceding claims wherein at least one side of the disc is convex.

5. The disc of any one of the preceding claims wherein both sides of the disc are convex.

6. A method of treating a human mouth by adhering in the mouth on or near a location needing treatment a convex lenticular shaped oral adhering disc, the method comprising:
(a) placing in the mouth an adhering disc comprising:
(1) a circumferential edge and two sides at least one of which sides has a convex surface and the disc has a thickness at its center greater than a thickness near its edge,
(2) at least one of which sides has an adhering surface comprising one or more binder ingredients;
and
(b) causing an adhesive side of the disc to adhere in the mouth on or near a location needing treatment by holding the disc in a desired location without movement.

7. The method of claim 6 wherein, when placed in a human mouth and not chewed, the disc does not disintegrate into more than one piece.

8. The method of claim 6 or 7 wherein, when placed in a human mouth and not chewed, the disc lasts for more than one hour before dissolving or eroding.

9. The method of any one of claims 6 to 8 wherein the disc is held in a desired location without movement for 10 to 40 seconds.

10. The method of any one of claims 6 to 9 wherein the treatment is for a lesion and the desired location is on or near the lesion.

11. The method of any one of claims 6 to 10 wherein the lesion is a mouth ulcer.

12. The method of any one of claims 6 to 11 wherein at least one side of the disc is convex.

13. The method of any one of claims 6 to 12 wherein both sides of the disc are convex.
